# FASCICULE DE BREVET EUROPEEN

(11) **EP 3 202 288 B1**
(45) Date de publication et mention de la délivrance du brevet: **17.10.2018**
(21) Numéro de dépôt: 17152966.2
(22) Date de dépôt: 25.01.2017
(51) Int. Cl.: A47G 1/20, F16B 15/00, B25C 9/00

(54) **PIÈCE D'ASSEMBLAGE A CLOUS INCLINÉS DE FIXATION ET OUTIL DE FIXATION D'UNE TELLE PIÈCE D'ASSEMBLAGE**
VERBINDUNGSTEIL MIT SCHRÄGEN BEFESTIGUNGSNÄGELN UND BEFESTIGUNGSWERKZEUG FÜR EIN SOLCHES VERBINDUNGSTEIL
ASSEMBLY PART WITH TILTED FASTENING NAILS AND TOOL FOR FASTENING SUCH AN ASSEMBLY PART

(30) Priorité: 26.01.2016 FR 1650639
(43) Date de publication de la demande: 09.08.2017
(73) Titulaire: NORAIL, 59360 Le Cateau Cambresis (FR)
(72) Inventeur: Colin, Emeric, 59221 Beauvin (FR); Gavid, Aurel, 65000 Tarbes (FR); Lorrillere, Emeric, 14000 Caen (FR); Eeckhoudt, Gilles, 59800 Lille (FR)
(74) Mandataire: Cabinet Rifflart Vandenbossche

(56) Documents cités:
- FR-A1- 2 612 224
- JP-A- S5 683 314
- US-A- 2 288 439
- US-A- 4 041 558

## Description

### Domaine technique

La présente invention se rapporte au domaine des accessoires à usage domestique tels qu'un crochet ou une patère permettant de suspendre un vêtement, une serviette et autres articles. Cet accessoire peut également consister en un support de penderie permettant la réception d'une extrémité de barre, une équerre permettant le support d'une étagère, voire tous autres accessoires. L'invention vise tout particulièrement à faciliter la fixation de tels accessoires à une paroi, notamment une paroi murale ou un plafond. L'invention porte également sur un outil de fixation d'un tel accessoire.

### Etat de la technique

Les accessoires tels que des crochets, des patères, des supports de penderies, des équerres d'étagères, voire tous autres accessoires à usage domestique, sont traditionnellement fixés à une paroi d'une habitation, notamment une paroi murale ou un plafond, aux moyens de vis et de chevilles assurant un ancrage dans la paroi. Pour cela, il convient de percer la paroi au moyen d'une perceuse et d'un forêt adapté, de mettre en place la cheville dans le trou de percement, puis d'ancrer la cheville dans la paroi, soit préalablement à la mise en place de la vis qui fixe l'accessoire soit au moment de la mise en place de cette vis. Il est donc nécessaire de disposer de l'outillage adéquat pour fixer de tels accessoires à une paroi. En outre, lors du retrait des accessoires, par exemple lors d'un changement de décor ou d'agencement d'une pièce de l'habitation, la paroi murale est endommagée de manière assez conséquente du fait de l'ancrage de la vis, nécessitant son arrachement de la paroi.

Il est également connu de fixer une pièce constituant ou recevant un tel accessoire, au moyen de plusieurs clous qui traversent l'épaisseur de ladite pièce et sont enfoncés dans la paroi avec des orientations ou inclinaisons différentes, de sorte à assurer l'ancrage de cette pièce. De telles conceptions apparaissent dans les demandes de brevet ou brevet publiés sous les numéros suivants: BE431800A, BE1012046A3, CA2000590A1, EP1267087A1, FR2612224A1, FR2687559A1, FR2702941A1, US2005035264A1, WO03054397A2, WO9420785A1. Lors du retrait de l'accessoire, il est généralement difficile d'accéder aux têtes des clous qui sont enfoncées dans la pièce, ce qui impose leur retrait en tirant directement sur ladite pièce. L'inclinaison des clous a pour incidence d'endommager la paroi lors de la traction exercée sur la pièce.

L'enfoncement des clous dans la paroi est généralement réalisé au moyen d'un marteau, lesdits clous étant enfoncés les uns après les autres. Les forces de frappe sur les têtes des clous au moyen du marteau sont différentes du fait des orientations différentes desdits clous. Lorsque l'installateur frappe sur le clou suivant ceux déjà enfoncés, il soumet la pièce à une force différente de celles exercées par les clous déjà enfoncés et retenant ladite pièce, ce qui a pour incidence d'élargir les trous de passage générés dans la paroi par ces clous enfoncés et de créer des jeux de montage. La paroi est donc endommagée et l'accessoire n'est pas convenablement ancré à la paroi.

Il est également connu des outils permettant d'enfoncer un clou sur une paroi pour fixer un accessoire. De tels outils comprennent généralement un corps permettant le maintien de l'outil et un piston monté à coulissement dans le corps d'une position de repos à une position de travail, et inversement. Ce piston débouche à l'extérieur du fond du corps de sorte à accéder à son extrémité proximale et, ainsi, à permettre la manipulation et l'activation du piston de la position repos à la position de travail, et inversement. Le corps est débouchant à son extrémité distale de sorte à réceptionner le clou. En outre, le piston comprend également une tête à son extrémité distale, qui vient percuter le clou lors du passage de la position repos à la position travail, pour son enfoncement selon une trajectoire perpendiculaire au plan d'appui sur la paroi. De telles conceptions apparaissent dans les demandes de brevet ou brevets publiés sous les numéros GB1067922A, US4299021A, US4655423A.

US4041558 divulgue un autre outil de fixation comprenant un corps configuré pour être maintenu et une tête de percussion étant configurée pour frapper un clou. Il est également connu la demande de brevet WO2010020951A1 qui divulgue un accessoire comportant un élément de support muni d'une pluralité de pointes parallèles entre-elles. Ces pointes sont configurées pour s'incurver progressivement durant leur enfoncement dans une paroi, ce qui permet fixer l'élément de support. Cette conception n'assure cependant pas un ancrage convenable, puisqu'un effort inverse à celui de l'enfoncement, tel que celui pouvant être généré par une charge, suffit à désengager les pointes qui tendent à revenir vers leur position initiale, parallèles entre-elles. Ce document WO2010020951A1 divulgue également un outil permettant la fixation d'un tel accessoire, ledit outil reprenant les caractéristiques précitées. Dans ce cas, toutefois, la tête du piston prend appui directement sur l'élément de support constituant une tête commune à toutes les pointes.

### Résumé de l'invention

La présente invention permet de pallier les inconvénients précités et a pour objectif principal de réduire l'endommagement de la paroi lors du montage et du démontage d'un tel accessoire.

A cet effet, l'invention concerne tout d'abord une pièce d'assemblage pour l'installation d'un accessoire tel qu'un crochet ou une patère permettant de suspendre un vêtement, une serviette et autres articles, un support de penderie permettant la réception d'une extrémité de barre, une équerre permettant le support d'une étagère, voire tous autres accessoires. Cette pièce d'assemblage est destinée à être fixée à une paroi, par exemple une paroi murale ou un plafond, selon l'accessoire. Cette pièce comprend un élément de support configuré pour maintenir ledit accessoire. L'élément de support peut constituer l'accessoire ou, préférentiellement, être conçu pour recevoir et maintenir un tel accessoire, comme cela apparaîtra plus en détail ci-après. Cet élément de support comprend une face d'appui destinée à venir au contact de la paroi. En outre, la pièce d'assemblage comprend au moins trois clous inclinés selon des orientations différentes, qui traversent l'élément de support. Ces clous sont destinés à être enfoncés dans la paroi pour la fixation de l'élément de support. De manière remarquable, l'élément de support comprend des moyens de rupture en autant de morceaux que de clous, configurés pour rendre indépendant chaque clou vis-à-vis des autres, de sorte à permettre leur retrait individuellement. Ainsi, lors du retrait de l'accessoire en vue d'un changement de décor ou d'agencement de la pièce, l'installateur casse l'élément de support en morceaux, ce qui permet de rendre indépendant les clous et de les retirer individuellement. Ce retrait individuel permet d'extraire chaque clou en exerçant une force dans le sens de l'orientation dudit clou, ce qui évite l'endommagement de la paroi.

Dans une réalisation de la pièce d'assemblage selon l'invention, les moyens de rupture sont constitués d'autant de fentes de rupture que de clous, lesdites fentes de rupture s'étendant de la périphérie de l'élément de support en direction son centre en formant des quartiers d'éléments de support. On pourrait prévoir d'autres variantes, par exemple un agencement de ces fentes de rupture dans le sens de l'épaisseur de l'élément de support.

Dans une réalisation de la pièce d'assemblage selon l'invention, les clous sont disposés sur les quartiers ou les morceaux. Ainsi, lors de la rupture de l'élément de support en quartiers ou en morceaux, chaque quartier ou morceau est dégagé simultanément au retrait du clou qu'il reçoit. On pourrait envisager une variante selon laquelle les clous seraient disposés entre les bords attenants entre les quartiers ou les morceaux, ce qui correspond à un agencement des clous au niveau des moyens de rupture.

Dans une réalisation de la pièce d'assemblage, l'élément de support comprend un trou traversant sur son épaisseur et les extrémités des fentes de rupture précitées sont avoisinantes à la paroi intérieure constituée par le trou. Cette conception facilite la rupture de la pièce d'assemblage.

Dans une réalisation de la pièce d'assemblage, les clous sont inclinés vers l'extérieur et ont leurs têtes qui se rejoignent vers un sommet. De préférence ce sommet est situé dans l'axe central de l'élément de support. D'autres orientations de clous peuvent être envisagées tout en prévoyant de tels moyens de rupture sur l'élément de support.

Dans une réalisation préférentielle, lorsqu'un trou traversant est prévu sur l'élément de la pièce d'assemblage et que les têtes des clous se rejoignent vers un sommet, la paroi intérieure du trou présente une inclinaison correspondant à l'inclinaison des clous. De préférence, l'élément de support à une forme circulaire et le trou est conique. La forme conique du trou permet de faciliter grandement la rupture de l'élément d'assemblage.

Dans une réalisation préférentielle, la pièce d'assemblage comprend trois clous. On peut envisager des variantes avec plus de trois clous, notamment quatre clous voire plus, selon la forme de l'élément de support.

Dans une réalisation de la pièce d'assemblage, l'élément de support est cylindrique et comprend des cannelures externes et une gorge externe circulaire. Ces éléments constituent des moyens de maintien destinés à coopérer avec d'autres moyens de maintien complémentaires, agencés sur un accessoire que ledit élément de support est destiné à recevoir.

L'invention concerne également un outil de fixation d'une pièce d'assemblage présentant les caractéristiques précitées. Cet outil de fixation comprend un corps configuré pour être maintenu avec une main et un piston monté à coulissement dans le corps pour translater d'une position de repos à une position de travail, et inversement. Le piston comprend une extrémité proximale débouchant à l'extérieur du fond du corps, de sorte à permettre la manipulation et l'activation du piston de la position repos à la position de travail, et inversement. Cette extrémité proximale peut comprendre un pommeau légèrement aplati configuré pour pousser ou frapper le piston afin de le déplacer en position de travail. En outre, le piston comprend une tête de percussion à son extrémité distale. Cette tête de percussion est configurée pour frapper ou pousser concomitamment les clous en exerçant des forces selon des trajectoires correspondant respectivement aux inclinaisons desdits clous sur l'élément de support de la pièce d'assemblage. Ainsi, la tête de percussion permet d'enfoncer les clous dans la paroi avec une répartition uniforme et concomitante des efforts sur la paroi, ce qui évite l'endommagement de la paroi et permet de conserver un parfait ancrage de la pièce d'assemblage sur cette paroi, sans jeu de fixation.

Dans une réalisation de l'outil de fixation, la tête de percussion a une forme conique configurée pour frapper simultanément les têtes des clous d'une pièce d'assemblage, lesdits clous étant inclinés vers l'extérieur et ayant leurs têtes qui se rejoignent vers un sommet. D'autres formes de tête de percussion peuvent être envisagées en fonction du nombre et de l'orientation des clous sur l'élément de support. Par exemple, la tête de percussion peut avoir une forme pyramidale lorsque l'élément de support reçoit quatre clous uniformément répartis et inclinés vers l'extérieur, avec leurs têtes qui se rejoignent vers un sommet.

Dans une réalisation de l'outil de fixation, le piston comprend une portion longitudinale conique et le fond du corps comprend un trou conique destiné à recevoir ladite portion conique. Ces formes coniques permettent de maintenir temporairement le piston en position de repos vis-à-vis du corps. Cela facilite la mise en place de l'élément de support sur le corps de l'outil, avant l'actionnement du piston dans la position de travail.

Dans une réalisation de l'outil de fixation, le corps comprend une extrémité distale munie d'un premier logement débouchant sur l'extérieur et configuré pour réceptionner l'élément de support avec les têtes des clous disposées du côté de la tête de percussion du piston.

Tel que précité, l'accessoire est configuré pour être reçu et maintenu par une pièce d'assemblage selon l'invention. L'accessoire comprend un second logement configuré pour réceptionner la pièce d'assemblage. De préférence, l'élément de support de la pièce d'assemblage est de forme cylindrique et le second logement est cylindrique. On pourrait envisager un élément de support de forme polygonale et un second logement de forme polygonale complémentaire. En outre, l'accessoire comprend des moyens de maintien en position sur ladite pièce d'assemblage. De préférence, ces moyens de maintien sur l'accessoire coopèrent avec des moyens de maintien complémentaires sur l'élément de support de la pièce d'assemblage. Cette conception permet de dissocier l'accessoire en tant que tel, qui a pour fonction d'accrocher un vêtement, de supporter une barre de penderie, de supporter une étagère, voire autre, de la pièce d'assemblage qui a pour fonction d'assurer l'ancrage à la paroi dudit accessoire qu'elle reçoit et maintient. Cela facilite la mise en oeuvre des moyens de rupture sur l'élément de support qui comprend une forme basique. Cela facilite également le positionnement des clous inclinés sur l'élément support et leur fixation à la paroi grâce à l'outil de fixation. On pourrait cependant envisager des variantes d'accessoires pouvant être intégrées directement à l'élément de support de la pièce d'assemblage, sans sortir du cadre de l'invention, par exemple pour des accessoires basics comme des pions de support d'un plateau d'angle agencé entre deux parois en angle.

Dans une réalisation préférentielle de l'accessoire, le second logement comprend des cannelures internes configurées pour s'engager dans les cannelures externes agencées sur l'élément de support de la pièce d'assemblage, telles que précitées. En outre, au moins une lumière est mise en oeuvre sur l'accessoire pour correspondre avec la gorge circulaire lorsque les cannelures internes et externes coopèrent. Ainsi les cannelures bloquent la rotation de l'accessoire vis-à-vis de l'élément support selon un axe perpendiculaire au plan de la paroi, et la correspondance de la lumière avec la gorge circulaire permet le positionnement d'une pièce de butée adaptée, par exemple une vis, une goupille ou une clé de butée, assurant l'arrêt en translation de l'accessoire vis-à-vis de l'élément de support, selon ledit axe.

Dans une réalisation, l'accessoire constitue un crochet, une patère, un support de penderie, ou un support en équerre. D'autres types d'accessoires restent envisageables sans sortir du cadre de l'invention.

L'invention concerne également un kit comportant au moins une pièce d'assemblage, un outil de fixation, et au moins un accessoire, qui présentent chacun les caractéristiques précitées contribuant à réduire l'endommagement de la paroi mural lors de l'installation de l'accessoire. De préférence le kit comprend autant de pièces d'assemblage que d'accessoires. Un tel kit permet au consommateur de disposer de tous les éléments nécessaires à l'installation des accessoires. On peut cependant envisager que le consommateur puisse acheter individuellement des pièces d'assemblage, par exemple lorsqu'il souhaite changer de position un accessoire, ou puisse acheter individuellement des accessoires, par exemple lorsqu'il souhaite remplacer à la même position sur la paroi un crochet par une patère, l'utilisateur pouvant réutiliser l'outil de fixation qu'il s'est déjà procuré avec le kit.

### Brève description des figures

Les caractéristiques et avantages de l'invention apparaîtront à la lecture de la description suivante s'appuyant sur des figures, parmi lesquelles :
- Les figures 1 à 4 illustrent quatre vues d'une pièce d'assemblage mettant en évidence l'élément de support et les inclinaisons des clous ;
- Les figures 5 à 8 illustrent quatre vues mettant en évidence un outil de fixation d'une pièce d'assemblage et les éléments constituant ledit outil de fixation ;
- Les figurent 9 et 10 illustrent un accessoire de type patère, destiné à être fixé à une paroi par le biais d'une pièce d'assemblage selon l'invention ;
- Les figurent 11 et 12 illustrent un accessoire de type crochet, destiné à être fixé à une paroi par le biais d'une pièce d'assemblage selon l'invention ;
- Les figurent 13 à 15 illustrent les éléments constitutif d'un accessoire de type équerre d'étagère, destinés à être fixés à une paroi par le biais d'une pièce d'assemblage selon l'invention ;
- Les figurent 16 et 17 illustrent un accessoire de type support de penderie, destiné à être fixé à une paroi par le biais d'une pièce d'assemblage selon l'invention.

### Description détaillée

Dans la suite de la description, les mêmes références sont utilisées pour les caractéristiques identiques ou similaires sur chaque variante de réalisation concernant les différents types d'accessoires.

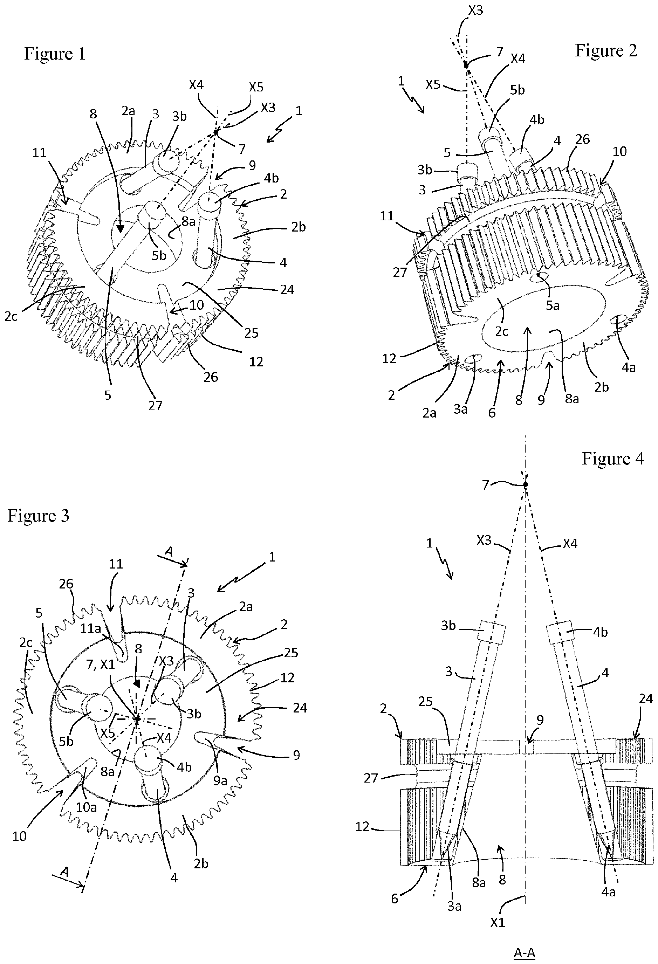

Sur les figures 1 à 4, la pièce d'assemblage 1 comprend un élément de support 2 et trois clous 3, 4, 5. L'élément de support 2 est destiné à être ancré sur une paroi au moyen des trois clous 3, 4, 5. L'élément de support 2 comprend une forme cylindrique, ce qui n'exclut pas la mise en oeuvre d'autres formes, notamment des formes polygonales. Cet élément de support 2 comprend une face d'appui 6 qui vient en contact avec le plan de la paroi lors de la fixation de la pièce d'assemblage 1. Les trois clous 3, 4, 5 traversent l'élément de support 2, comme l'illustrent notamment les figures 2 et 4. Ces trois clous 3, 4, 5 sont inclinés par rapport à l'axe longitudinal X1 passant par le centre de l'élément de support 2, de sorte que leurs pointes 3a, 4a, 5a s'éloignent vers l'extérieur et leurs têtes 3b, 4b, 5b se rejoignent vers un sommet 7 passant par l'axe longitudinal X1.

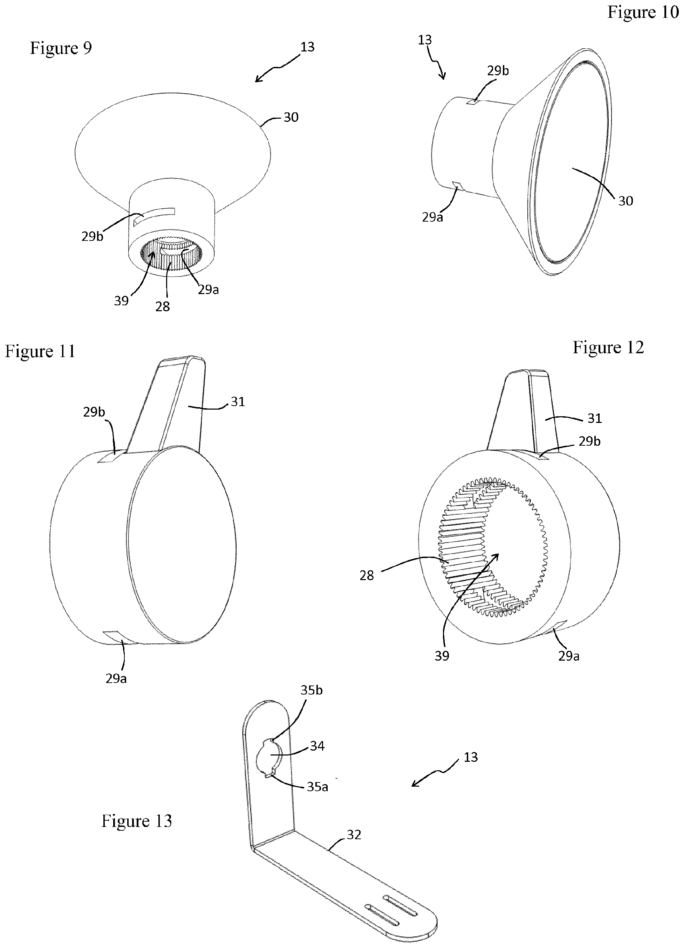

Tel qu'illustré sur les figures 1 à 4, l'élément de support 2 comprend un trou 8 traversant son épaisseur, et trois fentes de rupture 9, 10, 11 qui s'étendent radialement depuis la paroi externe 12 périphérique dudit élément de support 2. Le trou 8 est conique, l'angle du cône correspondant sensiblement à l'angle d'inclinaison des clous 3, 4, 5. En outre, les fonds 9a, 10a, 11a des trois fentes de rupture 9, 10, 11 sont inclinés, comme l'illustre la figure 3, ce qui permet auxdits fonds 9a, 10a, 11a de s'approcher au mieux de la paroi interne 8a du trou 8 conique. Cela facilite la rupture de l'élément de support 2 en morceaux, cette rupture pouvant être réalisée en utilisant un outil de forme plate, par exemple une tête plate d'un tournevis, ou un outil tranchant, par exemple un couteau à lame rétractable plus couramment appelé un « cutter ». L'élément de support 2 est de forme cylindrique. Lorsque l'élément de support 2 est rompu en morceaux selon les trois fentes de rupture 9, 10, 11, celui-ci se sépare en trois quartiers 2a, 2b, 2c qui reçoivent respectivement les trois clous 3, 4, 5. Cela permet de séparer physiquement chaque clou 3, 4, 5 pour les retirer individuellement lors du démontage d'un accessoire 13 rapporté sur ladite pièce d'assemblage 1, des variantes d'accessoires 13 étant illustrées sur les figures 9 à 17.

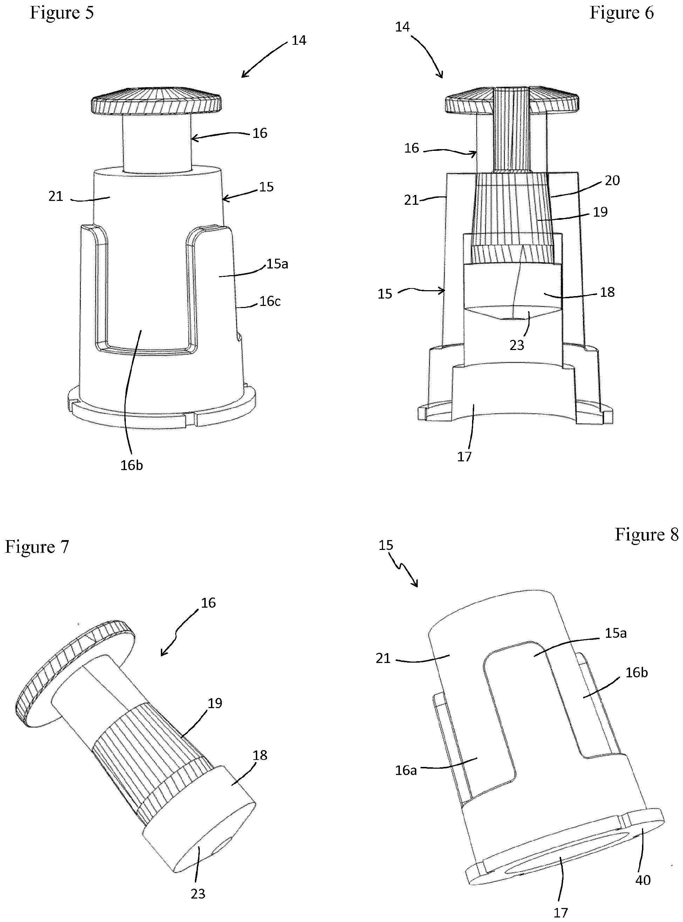

L'ancrage de la pièce d'assemblage 1 sur une paroi, par exemple une paroi murale ou un plafond, est réalisé au moyen d'un outil de fixation 14 illustré en figures 5 et 6. Tel qu'illustré au regard des figures 5 à 8, l'outil de fixation 14 comprend un corps 15 et un piston 16. Le corps 15 est cylindrique et creux, et il comprend sur sa périphérie externe 15a des évidements 16a, 16b 16c qui permettent de saisir convenablement l'outil de fixation 14 durant son utilisation.

Tel qu'illustré sur ces figures 5 à 8, le corps 15 comprend à son extrémité distale un logement 17 débouchant sur l'extérieur, de forme cylindrique configurée pour réceptionner l'élément de support 2, avec les têtes 3b, 4b, 5b des clous 3, 4, 5 disposées à proximité d'une tête de percussion 18 agencée à l'extrémité distale du piston 16, lorsque ledit piston se trouve dans une position de repos. Le piston 16 comprend une portion conique 19 qui vient se loger dans un trou conique 20 agencé dans le fond 21 du corps 15. Ce trou conique 20 sur le corps 15 permet de maintenir la portion conique 19 du piston 16, dans la position de repos, ce qui laisse toute liberté de manipulation durant la mise en place de la pièce d'assemblage 1 dans le logement 17 du corps 15.

Tel qu'illustré sur ces figures 5 à 8, le piston 16 passe au travers du fond 21 du corps 15 et comprend à son extrémité proximale un pommeau 22 de forme plus ou moins aplatie, ce qui permet de manipuler le piston 16 avec la main voire avec un marteau ou un maillet, pour déplacer le piston 16 vis-à-vis du corps 15, dans une position de travail selon laquelle la tête de percussion 18 frappe simultanément les têtes 3b, 4b, 5b des trois clous 3, 4, 5. Pour cela, la tête de percussion 18 comprend une face extrême 23 de forme conique, l'angle du cône étant agencé pour frapper les têtes 3b, 4b, 5b des clous 3, 4, 5 en exerçant des forces selon des trajectoires définies par les axes X3, X4, X5 d'inclinaison des clous 3, 4, 4, illustrés en figures 1 à 4. Cela permet d'enfoncer concomitamment les trois clous 3, 4, 5 sans endommager la paroi, permettant l'ancrage de l'élément de support 2. L'élément support 2 comprend sur sa face externe 24 un logement 25 qui permet la réception des têtes 3b, 4b, 5b des clous 3, 4, 5 en position enfoncée dans la paroi, ce qui évite leur dépassement de l'élément de support 2 lorsque celui-ci reçoit l'accessoire 13. Le logement 25 sur la face externe 24 de l'élément support 2 permet également à la tête de percussion 18 de l'outil de fixation 14, lorsque celle-ci vient enfoncer les clous 3, 4, 5, d'aller jusqu'au bout de sa course. Le trou 8 sur l'élément de support 2 permet l'introduction partielle de la face extrême 23 conique de la tête de percussion 18 dans la position de travail du piston 16, ce qui assure l'enfoncement en totalité des clous 3, 4, 5 dans l'élément de support 2. Durant la fixation de l'élément de support 2 au moyen de l'outil de fixation 14, la face extrémale 40 du corps 15 vient en appui sur la paroi.

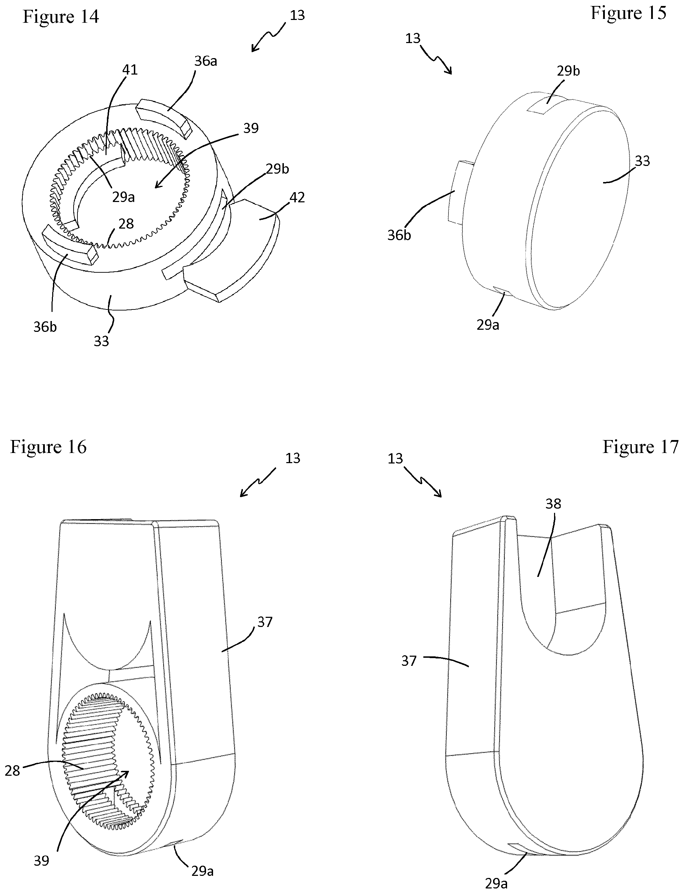

Tel qu'illustré en figures 1 à 4, l'élément de support 2 comprend des cannelures externes 26 et une gorge circulaire 27. Tels que l'illustrent les figures 9 à 17, montrant différentes conceptions d'un accessoire 13, ledit accessoire 13 comprend un logement 39 muni de cannelures internes 28 configurées pour être engagées dans les cannelures externes 26 de l'élément de support 2, ce qui permet l'emboîtement dudit accessoire 13 dans ledit élément de support 2 en bloquant la rotation selon l'axe X1. L'accessoire 13 comprend également une ou deux lumières 29a, 29b, selon la configuration de celui-ci. Ces lumières 29a, 29b correspondent avec la gorge circulaire 27 dans la position emboîtée précitée, ce qui permet l'introduction d'une ou deux pièces de butée du type clavettes 41, 42, illustrées en figure 14, dans la ou les lumières 29a, 29b afin de venir les loger dans la gorge circulaire 27, assurant ainsi le blocage en translation selon l'axe X1 de l'accessoire 13 vis-à-vis de l'élément de support 2.

Sur le mode de réalisation des figures 9 et 10, l'accessoire 13 constitue une patère 30. Sur le mode de réalisation des figures 11 et 12, l'accessoire 13 constitue un crochet 31. Sur le mode de réalisation des figures 13 à 15, l'accessoire 13 est constitué d'une équerre 32 et d'un élément de maintien 33 de cette équerre 32. L'équerre 32 comprend un orifice 34 muni de deux encoches 35a, 35b. L'orifice 34 est emboîté sur l'élément de support 2, puis l'élément de maintien 33 est assemblé avec ledit élément de support 2. Cet élément de maintien 33 comprend deux dents 36a, 36b qui viennent se loger respectivement dans les deux encoches 35a, 35b dans la position d'assemblage précitée. Sur le mode de réalisation des figures 16 et 17, l'accessoire 13 constitue un support de penderie 37 qui comprend une encoche 38 permettant la réception d'une extrémité d'une barre (non illustrée).

La description détaillée qui précède d'un mode de réalisation particulier de l'invention n'a aucun caractère limitatif. Bien au contraire, elle a pour objectif d'ôter toute éventuelle imprécision quant à sa portée. Ainsi, de nombreuses variantes pourront être envisagées dans le cadre de l'invention telle que revendiquée, notamment quant à la forme de l'élément de support 2, le nombre et les inclinaisons des clous 3, 4, 5, la mise en oeuvre des moyens de maintien entre l'élément de support 2 et l'accessoire 13, et la forme de la face extrême 23 sur la tête de percussion 18 du piston 16, laquelle dépend notamment du nombre et de l'inclinaison des clous 3, 4, 5.

Concernant les moyens de maintien sur l'élément de support 2 et ceux sur l'accessoire 13, il est notamment possible de remplacer les cannelures externes 26 et la gorge circulaire 27 sur cet élément de support 2 par un filetage et de remplacer les cannelures internes 28 et les deux lumières 29a, 29b par un taraudage dans le logement 39. Ainsi l'accessoire 13 se visse sur l'élément de support 2 pour son maintien en position.

Il devra être observé que cette description détaillée porte sur un exemple de réalisation particulier de la présente invention, mais qu'en aucun cas cette description ne revêt un quelconque caractère limitatif à l'objet de l'invention ; bien au contraire, elle a pour objectif d'ôter toute éventuelle imprécision ou toute mauvaise interprétation des revendications qui suivent.

Il devra également être observé que les signes de références mis entre parenthèses dans les revendications qui suivent ne présentent en aucun cas un caractère limitatif ; ces signes ont pour seul but d'améliorer l'intelligibilité et la compréhension des revendications qui suivent ainsi que la portée de la protection recherchée.

## Revendications

1. Pièce d'assemblage (1) pour l'installation d'accessoire (13), destinée à être fixée à une paroi, ladite pièce d'assemblage (1) comprenant un élément de support (2) configuré pour maintenir ledit accessoire, ledit élément de support comprenant une face d'appui (6) destinée à venir au contact de la paroi, ladite pièce d'assemblage comprenant au moins trois clous (3, 4, 5) inclinés selon des orientations différentes qui traversent l'élément de support, lesdits clous étant destinés à être enfoncés dans la paroi pour la fixation de l'élément de support, **caractérisé en ce que** ledit élément de support comprend des moyens de rupture (9, 10, 11) en autant de morceaux (2a, 2b, 2c) que de clous, configurés pour rendre indépendant chaque clou vis-à-vis des autres, de sorte à permettre leur retrait individuellement.

2. Pièce d'assemblage (1) selon la revendication 1, dans laquelle les moyens de rupture sont constitués d'autant de fentes de rupture (9, 10, 11) que de clous (3, 4, 5), lesdites fentes de rupture s'étendant de la périphérie (12) de l'élément de support (2) en direction son centre en formant des quartiers (2a, 2b, 2c).

3. Pièce d'assemblage (1) selon la revendication 2, dans laquelle les clous (3, 4, 5) sont disposés sur les quartiers (2a, 2b, 2c).

4. Pièce d'assemblage (1) selon l'une des revendications 2 ou 3, dans laquelle l'élément de support (2) comprend un trou (8) traversant son épaisseur, les extrémités (9a, 10a, 11a) des fentes (9, 10, 11) étant avoisinantes à la paroi intérieure (8a) constituée par le trou (8).

5. Pièce d'assemblage (1) selon l'une des revendications 1 à 4, dans laquelle les clous (3, 4, 5) sont inclinés vers l'extérieur et ont leurs têtes (3b, 4b, 5b) qui se rejoignent vers un sommet (7).

6. Pièce d'assemblage (1) selon la revendication 5 rattachée à la revendication 4, dans laquelle la paroi intérieure (8a) a une inclinaison correspondant à l'inclinaison des clous (3, 4, 5).

7. Pièce d'assemblage (1) selon l'une des revendications 1 à 6, laquelle comprend trois clous (3, 4, 5).

8. Pièce d'assemblage (1) selon l'une des revendications 1 à 7, dans laquelle l'élément de support (2) est cylindrique et comprend des cannelures externes (26) et une gorge externe circulaire (27).

9. Outil de fixation (14) d'une pièce d'assemblage (1) selon l'une des revendications 1 à 8, comprenant un corps (15) configuré pour être maintenu et un piston (16) monté à coulissement dans le corps d'une position de repos à une position de travail, et inversement, le piston comprenant une extrémité proximale débouchant à l'extérieur du fond (21) du corps de sorte à permettre la manipulation et l'activation du piston de la position repos à la position de travail, et inversement, **caractérisé en ce que** le piston comprend une tête de percussion (18) à son extrémité distale, ladite tête de percussion étant configurée pour frapper concomitamment les clous (3, 4, 5) en exerçant des forces selon des trajectoires (X3, X4, X5) correspondant respectivement aux inclinaisons desdits clous.

10. Outil de fixation (14) selon la revendication 9, dans lequel la tête de percussion (18) a une forme conique configurée pour frapper les têtes (3b, 4b, 5b) des clous (3, 4, 5), lesdits clous étant inclinés vers l'extérieur et ayant leurs têtes qui se rejoignent vers un sommet (7).

11. Outil de fixation (14) selon l'une des revendications 9 ou 10, dans lequel le piston (16) comprend une portion longitudinale conique (19) et le fond (21) du corps (15) comprend un trou conique (20) destiné à recevoir ladite portion conique (19), lesdites formes coniques permettant de maintenir temporairement le piston en position de repos.

12. Outil de fixation (14) selon l'une des revendications 9 à 11, dans lequel le corps (15) comprend une extrémité distale munie d'un premier logement (17) débouchant sur l'extérieur et configuré pour réceptionner l'élément de support (2) avec les têtes (3b, 4b, 5b) des clous (3, 4, 5) disposées du côté de la tête de percussion (18) du piston (16).

13. Kit comportant au moins une pièce d'assemblage (1) selon l'une des revendications 1 à 8, un outil de fixation (14) selon l'une des revendications 10 à 12, et au moins un accessoire (13) comprenant un second logement (39) configuré pour réceptionner la pièce d'assemblage (1) et des moyens de maintien (28, 29a, 29b) en position sur ladite pièce d'assemblage (1).

14. Kit selon la revendication 13, dans lequel le second logement (39) comprend des cannelures internes (28) configurées pour s'engager dans les cannelures externes (26) agencées sur l'élément de support (2) de la pièce d'assemblage (1) selon la revendication 8, au moins une lumière (29a, 29) étant mise en oeuvre sur ledit accessoire pour correspondre avec la gorge circulaire (27) lorsque les cannelures internes et externes coopèrent.

15. Kit selon l'une des revendications 13 ou 14, dans lequel l'accessoire (13) constitue un crochet (31), une patère (30), un support de penderie (37), ou un support en équerre (32,33).

## Patentansprüche

1. Verbindungsteil (1) zum Installieren eine Zubehörs (13), das dazu bestimmt ist, an einer Wand fixiert zu werden, wobei das Teil ein Trägerelement (2) umfasst, das konfiguriert ist, um das Zubehör festzuhalten, wobei das Trägerelement eine Auflagefläche (6) umfasst, die dazu bestimmt ist, in Kontakt mit der Wand zu treten, wobei das Verbindungsteil mindestens drei in unterschiedliche Ausrichtungen geneigte Nägel (3, 4, 5) umfasst, die durch das Trägerelement führen, wobei die Nägel dazu bestimmt sind, zur Fixierung des Trägerelements in die Wand eingeschlagen zu werden, **dadurch gekennzeichnet, dass** das Trägerelement Bruchmittel (9, 10, 11) in ebenso vielen Stücken (2a, 2b, 2c) wie Nägel umfasst, die konfiguriert sind, um jeden Nagel gegenüber den anderen jeweils unabhängig zu machen, um es zu ermöglichen, diese einzeln herauszuziehen.

2. Verbindungsteil (1) nach Anspruch 1, wobei die Bruchmittel aus ebenso vielen Bruchschlitzen (9, 10, 11) wie Nägeln (3, 4, 5) gebildet sind, wobei sich die Bruchschlitze vom Umfang (12) des Trägerelements (2) in Richtung seiner Mitte erstrecken, und dabei Spalten (2a, 2b, 2c) bilden.

3. Verbindungsteil (1) nach Anspruch 2, wobei die Nägel (3, 4, 5) auf den Spalten (2a, 2b, 2c) angeordnet sind.

4. Verbindungsteil (1) nach einem der Ansprüche 2 oder 3, wobei das Trägerelement (2) ein Loch (8) umfasst, das durch seine Dicke führt, wobei die Enden (9a, 10a, 11a) der Schlitze (9, 10, 11) an die Innenwand (8a) angrenzend sind, die durch das Loch (8) gebildet wird.

5. Verbindungsteil (1) nach einem der Ansprüche 1 bis 4, wobei die Nägel (3, 4, 5) nach außen geneigt sind und deren Köpfe (3b, 4b, 5b) in einem Scheitelpunkt (7) zusammenlaufen.

6. Verbindungsteil (1) nach Anspruch 5 in Verbindung mit Anspruch 4, wobei die Innenwand (8a) eine Neigung aufweist, die der Neigung der Nägel (3, 4, 5) entspricht.

7. Verbindungsteil (1) nach einem der Ansprüche 1 bis 6, welches drei Nägel (3, 4, 5) umfasst.

8. Verbindungsteil (1) nach einem der Ansprüche 1 bis 7, wobei das Trägerelement (2) zylindrisch ist und externe Rillen (26) und eine externe kreisförmige Nut (27) umfasst.

9. Fixierwerkzeug (14) für ein Verbindungsteil (1) nach einem der Ansprüche 1 bis 8, umfassend einen Korpus (15), der konfiguriert ist, um festgehalten zu werden, und einen Kolben (16), der in dem Korpus von einer Ruheposition in eine Arbeitsposition, und umgekehrt, gleitend montiert ist, wobei der Kolben ein proximales Ende, das außerhalb des Bodens (21) des Korpus mündet, umfasst, um die Handhabung und die Aktivierung des Kolbens von der Ruheposition in die Arbeitsposition, und umgekehrt, zu ermöglichen, **dadurch gekennzeichnet, dass** der Kolben einen Schlagkopf (18) an seinem distalen Ende umfasst, wobei der Schlagkopf konfiguriert ist, um gleichzeitig auf die Nägel (3, 4, 5) zu schlagen und Kräfte entlang der Bewegungsbahnen (X3, X4, X5) auszuüben, die jeweils den Neigungen der Nägel entsprechen.

10. Fixierwerkzeug (14) nach Anspruch 9, wobei der Schlagkopf (18) eine kegelige Form aufweist, die konfiguriert ist, um auf die Köpfe (3b, 4b, 5b) der Nägel (3, 4, 5) zu schlagen, wobei die Nägel nach außen geneigt sind und deren Köpfe in einem Scheitelpunkt (7) zusammenlaufen.

11. Fixierwerkzeug (14) nach einem der Ansprüche 9 oder 10, wobei der Kolben (16) einen kegeligen Längsabschnitt (19) umfasst und der Boden (21) des Korpus (15) ein kegeliges Loch (20) umfasst, das dazu bestimmt ist, den kegeligen Abschnitt (19) aufzunehmen, wobei es die kegeligen Formen ermöglichen, den Kolben vorübergehend in der Ruheposition festzuhalten.

12. Fixierwerkzeug (14) nach einem der Ansprüche 9 bis 11, wobei der Korpus (15) ein distales Ende umfasst, das mit einer ersten Aufnahme (17) versehen ist, die nach außen mündet und konfiguriert ist, um das Trägerelement (2) mit den Köpfen (3b, 4b, 5b) der Nägel (3, 4, 5) aufzunehmen, die auf Seiten des Schlagkopfes (18) des Kolbens (16) angeordnet sind.

13. Bausatz, mindestens ein Verbindungsteil (1) nach einem der Ansprüche 1 bis 8, ein Fixierwerkzeug (14) nach einem der Ansprüche 10 bis 12, und mindestens ein Zubehör (13) aufweisend, das eine zweite Aufnahme (39) umfasst, die konfiguriert ist, um das Verbindungsteil (1) und Haltemittel (28, 29a, 29b) in Position auf dem Verbindungsteil (1) aufzunehmen.

14. Bausatz nach Anspruch 13, wobei die zweite Aufnahme (39) interne Rillen (28) umfasst, die konfiguriert sind, um in die externen Rillen (26) eingeführt zu werden, die an dem Trägerelement (2) des Verbindungsteils (1) nach Anspruch 8 angeordnet sind, wobei mindestens ein Langloch (29a, 29) an dem Zubehör zur Anwendung kommt, um der kreisförmigen Nut (27) zu entsprechen, wenn die internen und die externen Rillen zusammenwirken.

15. Bausatz nach einem der Ansprüche 13 oder 14, wobei das Zubehör (13) einen Haken (31), einen Kleiderhaken (30), einen Kleiderstangenträger (37), oder einen Winkelträger (32,33) bildet.

## Claims

1. Assembly part (1) for the installation of an accessory (13), intended to be fastened to a wall, said part comprising a support element (2) configured for maintaining said accessory, said support element comprising a bearing face (6) intended to come into contact with the wall, said assembly part comprising at least three nails (3, 4, 5) inclined according to different orientations that pass through the support element, said nails being intended to be driven into the wall for the fastening of the support element, **characterised in that** said support element comprises means for breaking (9, 10, 11) into as many pieces (2a, 2b, 2c) as there are nails, configured to render each nail independent with respect to the others, in such a way as to allow for the withdrawal thereof individually.

2. Assembly part (1) according to claim 1, wherein the means for breaking are comprised of as many slots for breaking (9, 10, 11) as there are nails (3, 4, 5), said slots for breaking extending from the periphery (12) of the support element (2) in the direction of its centre by forming quarters (2a, 2b, 2c).

3. Assembly part (1) according to claim 2, wherein the nails (3, 4, 5) are arranged on the quarters (2a, 2b, 2c).

4. Assembly part (1) according to one of claims 2 or 3, wherein the support element (2) comprises a hole (8) passing through its thickness, with the ends (9a, 10a, 11a) of the slots (9, 10, 11) being next to the inner wall (8a) formed by the hole (8).

5. Assembly part (1) according to one of claims 1 to 4, wherein the nails (3, 4, 5) are inclined outwards and have their heads (3b, 4b, 5b) which come together towards an apex (7).

6. Assembly part (1) according to claim 5 attached to claim 4, wherein the inner wall (8a) has an inclination that corresponds to the inclination of the nails (3, 4, 5).

7. Assembly part (1) according to one of claims 1 to 6, which comprises three nails (3, 4, 5).

8. Assembly part (1) according to one of claims 1 to 7, wherein the support element (2) is cylindrical and comprises external splines (26) and external circular groove (27).

9. Tool for fastening (14) an assembly part (1) according to one of claims 1 to 8, comprising a body (15) configured to be maintained and a piston (16) slidably mounted in the body from a rest position to a working position, and inversely, with the piston comprising a proximal end opening outside of the bottom (21) of the body in such a way as to allow for the manipulation and the activation of the piston from the rest position to the working position, and inversely, **characterised in that** the piston comprises a percussion head (18) at its distal end, said percussion head being configured to simultaneously strike the nails (3, 4, 5) by exerting forces according to trajectories (X3, X4, X5) that respectively correspond to the inclinations of said nails.

10. Tool for fastening (14) according to claim 9, wherein the percussion head (18) has a tapered shape configured to strike the heads (3b, 4b, 5b) of the nails (3, 4, 5), said nails being inclined outwards and having their heads that come together towards an apex (7).

11. Tool for fastening (14) according to one of claims 9 or 10, wherein the piston (16) comprises a tapered longitudinal portion (19) and the bottom (21) of the body (15) comprises a tapered hole (20) intended to receive said tapered portion (19), said tapered shapes making it possible to temporarily maintain the piston in the rest position.

12. Tool for fastening (14) according to one of claims 9 to 11, wherein the body (15) comprises a distal end provided with a first housing (17) opening outwards and configured to receive the support element (2) with the heads (3b, 4b, 5b) of the nails (3, 4, 5) arranged on the side of the percussion head (18) of the piston (16).

13. Kit comprising at least one assembly part (1) according to one of claims 1 to 8, a tool for fastening (14) according to one of claims 10 to 12, and at least one accessory (13) comprising a second housing (39) configured to receive the assembly part (1) and means for maintaining (28, 29a, 29b) in position on said assembly part (1).

14. Kit according to claim 13, wherein the second housing (39) comprises internal splines (28) configured to engage into the external splines (26) arranged on the support element (2) of the assembly part (1) according to claim 8, at least one hole (29a, 29) being implemented on said accessory to correspond with the circular groove (27) when the internal and external splines cooperate.

15. Kit according to one of claims 13 or 14, wherein the accessory (13) forms a hook (31), a peg (30), a wardrobe support (37), or a square bracket (32, 33).
